# EUROPEAN PATENT SPECIFICATION

(11) **EP 2 303 399 B1**
(45) Date of publication and mention of the grant of the patent: **23.08.2017**
(21) Application number: 09735094.6
(22) Date of filing: 24.04.2009
(51) Int. Cl.: A61N 1/05, A61N 1/36

(54) **SIMPLY SUPPORTED NEURAL STIMULATION ELECTRODE ARRAY FOR APPLYING PRESSURE ON NEURAL TISSUE**
EINFACH GESTÜTZTE NERVENSTIMULATIONS-ELEKTRODENANORDNUNG ZUM AUFBRINGEN VON DRUCK AUF NERVENGEWEBE
RÉSEAU D ÉLECTRODES DE STIMULATION NEURALE SIMPLE POUR L APPLICATION DE PRESSION SUR UN TISSU NEURONALE

(30) Priority: 25.04.2008 US 48120
(43) Date of publication of application: 06.04.2011
(73) Proprietor: Second Sight Medical Products, Inc., Sylmar, CA 91342 (US)
(72) Inventor: GREENBERG, Robert, J., Los Angeles CA 90068 (US); KHALDI, Mohamed, Los Angeles CA 90049 (US); LITTLE, James, S., Saugus CA 91350 (US); NEYSMITH, Jordan, M., Pasadena CA 91101 (US); TALBOT, Neil, Hamilton, La Crescenta CA 91214 (US)
(74) Representative: Carter, Stephen John
(86) International application number: PCT/US2009/041701
(87) International publication number: WO 2009/132296

(56) References cited:
- WO-A-2007/149571
- US-A- 5 575 813

## Description

### Field of the Invention

The present invention is generally directed to neural stimulation and more specifically to an improved electrode array for neural stimulation.

### Background of the Invention

In 1755 LeRoy passed the discharge of a Leyden jar through the orbit of a man who was blind from cataract and the patient saw "flames passing rapidly downwards." Ever since, there has been a fascination with electrically elicited visual perception. The general concept of electrical stimulation of retinal cells to produce these flashes of light or phosphenes has been known for quite some time. Based on these general principles, some early attempts at devising prostheses for aiding the visually impaired have included attaching electrodes to the head or eyelids of patients. While some of these early attempts met with some limited success, these early prosthetic devices were large, bulky and could not produce adequate simulated vision to truly aid the visually impaired.

In the early 1930's, Foerster investigated the effect of electrically stimulating the exposed occipital pole of one cerebral hemisphere. He found that, when a point at the extreme occipital pole was stimulated, the patient perceived a small spot of light directly in front and motionless (a phosphene). Subsequently, Brindley and Lewin (1968) thoroughly studied electrical stimulation of the human occipital (visual) cortex. By varying the stimulation parameters, these investigators described in detail the location of the phosphenes produced relative to the specific region of the occipital cortex stimulated. These experiments demonstrated: (1) the consistent shape and position of phosphenes; (2) that increased stimulation pulse duration made phosphenes brighter; and (3) that there was no detectable interaction between neighboring electrodes which were as close as 2.4 mm apart.

As intraocular surgical techniques have advanced, it has become possible to apply stimulation on small groups and even on individual retinal cells to generate focused phosphenes through devices implanted within the eye itself. This has sparked renewed interest in developing methods and apparatus to aid the visually impaired. Specifically, great effort has been expended in the area of intraocular retinal prosthesis devices in an effort to restore vision in cases where blindness is caused by photoreceptor degenerative retinal diseases; such as retinitis pigmentosa and age related macular degeneration which affect millions of people worldwide.

Neural tissue can be artificially stimulated and activated by prosthetic devices that pass pulses of electrical current through electrodes on such a device. The passage of current causes changes in electrical potentials across visual neuronal membranes, which can initiate visual neuron action potentials, which are the means of information transfer in the nervous system.

Based on this mechanism, it is possible to input information into the nervous system by coding the sensory information as a sequence of electrical pulses which are relayed to the nervous system via the prosthetic device. In this way, it is possible to provide artificial sensations including vision.

One typical application of neural tissue stimulation is in the rehabilitation of the blind. Some forms of blindness involve selective loss of the light sensitive transducers of the retina. Other retinal neurons remain viable, however, and may be activated in the manner described above by placement of a prosthetic electrode device on the inner (toward the vitreous) retinal surface (epiretinal). This placement must be mechanically stable, minimize the distance between the device electrodes and the visual neurons, control the electronic field distribution and avoid undue compression of the visual neurons.

In 1986, Bullara (US Pat. No. 4,573,481) patented an electrode assembly for surgical implantation on a nerve. The matrix was silicone with embedded iridium electrodes. The assembly fit around a nerve to stimulate it.

Dawson and Radtke stimulated cat's retina by direct electrical stimulation of the retinal ganglion cell layer. These experimenters placed nine and then fourteen electrodes upon the inner retinal layer (i.e., primarily the ganglion cell layer) of two cats. Their experiments suggested that electrical stimulation of the retina with 30 to 100 µA current resulted in visual cortical responses. These experiments were carried out with needle-shaped electrodes that penetrated the surface of the retina (see also US Pat. No. 4,628,933 to Michelson).

The Michelson '933 apparatus includes an array of photosensitive devices on its surface that are connected to a plurality of electrodes positioned on the opposite surface of the device to stimulate the retina. These electrodes are disposed to form an array similar to a "bed of nails" having conductors which impinge directly on the retina to stimulate the retinal cells. US Patent 4,837,049 to Byers describes spike electrodes for neural stimulation. Each spike electrode pierces neural tissue for better electrical contact. US Patent 5,215,088 to Norman describes an array of spike electrodes for cortical stimulation. Each spike pierces cortical tissue for better electrical contact.

The art of implanting an intraocular prosthetic device,to electrically stimulate the retina was advanced with the introduction of retinal tacks in retinal surgery. De Juan, et al. at Duke University Eye Center inserted retinal tacks into retinas in an effort to reattach retinas that had detached from the underlying choroid, which is the source of blood supply for the outer retina and thus the photoreceptors. See, e.g., E. de Juan, et al., 99 Am. J. Ophthalmol. 272 (1985). These retinal tacks have proved to be biocompatible and remain embedded in the retina, and choroid/sclera, effectively pinning the retina against the choroid and the posterior aspects of the globe. Retinal tacks are one way to attach a retinal electrode array to the retina. US Patent 5,109,844 to de Juan describes a flat electrode array placed against the retina for visual stimulation. US Patent 5,935,155 to Humayun describes a retinal prosthesis for use with the flat retinal array described in de Juan.

US patent 5,575,813, Edell describes a cantilever approach to attaching an electrode array to a retina. Edell describes a fundamentally flat array attached at one end acting as a cantilever. This applied uneven forces on the retina. It will apply greater force closer to the tack and greater force along the edges of the array.
WO 2006/116765 describes a flexible circuit electrode array.

### Summary of the Invention

The present invention is an electrode array for neural stimulation as set out in the appended claims. The invention will be best understood from the following description when read in conjunction with the accompanying drawings.

### Brief Description of the Drawings

**FIG. 1** is a perspective view of the preferred electrode array for retinal stimulation.
**FIG. 2** is a cross sectional view of the preferred electrode array for retinal stimulation.
**FIG. 3** depicts the top view of the flexible circuit array being enveloped within an insulating material.
**FIG. 4** depicts a cross-sectional view of the flexible circuit array being enveloped within an insulating material, a tack hole and a heel to apply force across the a fulcrum like point created by the tack.
**FIG. 5** depicts a cross-sectional view of the flexible circuit array being enveloped within an insulating material with open electrodes and the material between the electrodes.
**FIG. 6** depicts a cross-sectional view of the flexible circuit array being enveloped within an insulating material with an open window exposing the electrodes.
**FIG. 7** depicts a cross-sectional view of the flexible circuit array being enveloped within an insulating material formed in a spherical shape, except for a heel which is formed deviating from the primary curvature to apply additional pressure on the electrode region across from the fulcrum like point (tack location).
**FIG. 8** depicts a cross-sectional view of the prosthesis shown inside of the eye with an angle in the fold of the flexible circuit cable and a fold between the circuit electrode array and the flexible circuit cable.
**FIG. 9** depicts a cross-sectional view of the flexible circuit array illustrating two alternative embodiments.
**FIG. 10** depicts the top view of the flexible circuit array being enveloped within an insulating material.
**FIG. 11** depicts a cross-sectional view of the flexible circuit array being enveloped within an insulating material.
**FIG. 12** depicts a cross-sectional view of the flexible circuit array being enveloped within an insulating material with open electrodes and the material between the electrodes.
**FIG. 13** depicts a cross-sectional view of the flexible circuit array being enveloped within an insulating material with an open window exposing the electrodes.
**FIG. 14** depicts a cross-sectional view of the flexible circuit array being enveloped within an insulating material with electrodes on the surface of the material inside the eye with an angle in the fold of the flexible circuit cable and a fold between the circuit electrode array and the flexible circuit cable.
**FIG. 15** depicts a side view of the enlarged portion of the flexible circuit array being enveloped within an insulating material with electrodes on the surface of the material in the eye and contacting the retina.
**FIG. 16** is a perspective view of the implanted portion of the preferred retinal prosthesis.
**FIG. 17** is a side view of the implanted portion of the preferred retinal prosthesis showing the fan tail in more detail.
**FIG. 18** is a view of the completed package attached to an electrode array.
**FIG. 19** is a cross-section of the package.

### Detailed Description of the Preferred Embodiments

The following description is of the best mode presently contemplated for carrying out the invention. This description is not to be taken in a limiting sense, but is made merely for the purpose of describing the general principles of the invention. The scope of the invention should be determined with reference to the claims.

Polymer materials are useful as electrode array bodies for neural stimulation. They are particularly useful for retinal stimulation to create artificial vision, cochlear stimulation to create artificial hearing, or cortical stimulation for many purposes. Regardless of which polymer is used, the basic construction method is the same. A layer of polymer is laid down, commonly by some form of chemical vapor deposition, spinning, meniscus coating or casting. A layer of metal, preferably platinum, is applied to the polymer and patterned to create electrodes and leads for those electrodes. Patterning is commonly done by photolithographic methods. A second layer of polymer is applied over the metal layer and patterned to leave openings for the electrodes, or openings are created later by means such as laser ablation. Hence the array and its supply cable are formed of a single body. Additionally, multiple alternating layers of metal and polymer may be applied to obtain more metal traces within a given width.

The pressure applied against the retina, or other neural tissue, by an electrode array is critical. Too little pressure causes increased electric field dispersion between the array and retina. Too much pressure may block blood flow causing retinal ischemia and hemorrhage. Pressure on the neural retina may also block axonal flow or cause neuronal atrophy. Common flexible circuit fabrication techniques such as photolithography generally require that a flexible circuit electrode array be made flat. Since the retina is spherical, a flat array will necessarily apply more pressure near its edges, than at its center. Further, the edges of a flexible circuit polymer array may be quite sharp and cut the delicate retinal tissue. With most polymers, it is possible to curve them when heated in a mold. By applying the right amount of heat to a completed array, a curve can be induced that matches the curve of the retina. With a thermoplastic polymer such as liquid crystal polymer, it may be further advantageous to repeatedly heat the flexible circuit in multiple molds, each with a decreasing radius. Further, it is advantageous to encase the polymer within a molded soft polymer, such as silicone which holds the array in a spherical shape. Particularly, it is advantageous to add material that is more compliant than the polymer used for the flexible circuit array.

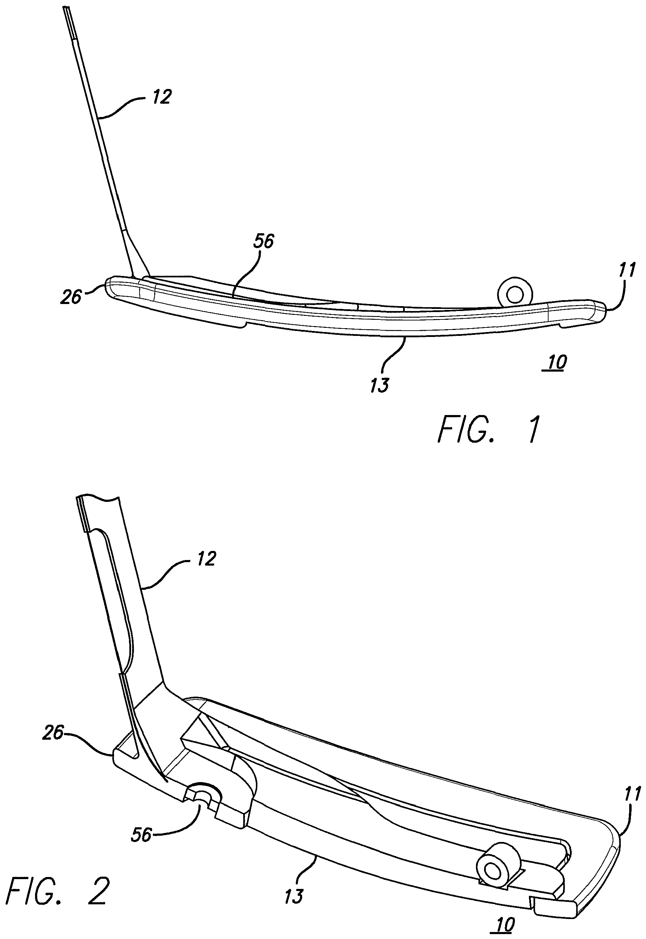

**Fig. 1** shows a side profile of the preferred simply supported electrode array for neural stimulation. The electrode array **10** includes an electrode array field including electrode **13** within a flexible body **11.** The array **10** is attached to neural tissue at an attachment point **56.** Preferably the attachment point is a hole suitable to receive a tack (not shown). Opposite the attachment point **56** is a heel **26.** The heel does not include electrodes but acts as a lever against a fulcrum at the attachment point. As the array is implanted pressure of the tissue against the heel **26** causes a pressure on the array field against the neural tissue. The electrode array further includes a cable **12** for supplying stimulation signals to the array **10.** In the preferred embodiment the cable **12** exits the array **10** in the heel **26.** However, the array cable could exit the array **10** at any point. The array cable **12** is preferably sufficiently flexible to not impart any force on the array field.

**Fig. 2** shows a cross sectional perspective view of the preferred simply supported electrode array for neural stimulation. **Figure 2** shows the hole used as attachment point **56.** The force applied to the array **10** by the heel **26** may be modified by many methods including varying the contour, thickness, stiffness, or second moment area of the material in the heel **26.**

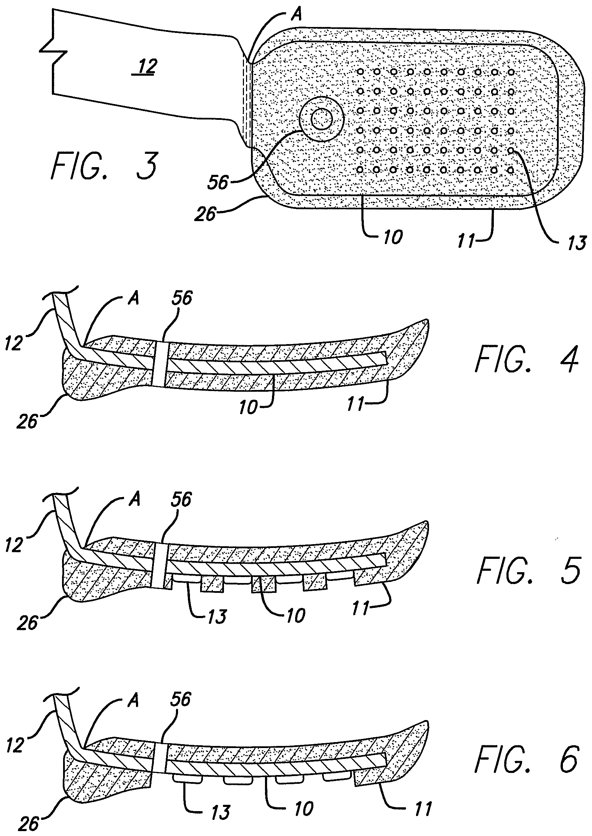

**Fig. 3** depicts the top view of the flexible circuit array **10** being enveloped within an insulating material **11.** The electrode array **10** comprises oval-shaped electrode array body **10,** a plurality of electrodes **13** made of a conductive material, such as platinum or one of its alloys, but that can be made of any conductive biocompatible material such as iridium, iridium oxide or titanium nitride. The electrode array **10** is enveloped within an insulating material **11** that is preferably silicone. "Oval-shaped" electrode array body means that the body may approximate either a square or a rectangle shape, but where the corners are rounded. This shape of an electrode array is described in the U.S. Patent Application No. 20020111658, entitled "Implantable retinal electrode array configuration for minimal retinal damage and method of reducing retinal stress" and No. 20020188282, entitled "Implantable drug delivery device" to Robert J. Greenberg et al.

The insulating material body **11** is made of a soft material that is compatible with the electrode array body **10.** In a preferred embodiment the body **11** is made of silicone having hardness of about 50 or less on the Shore A scale as measured with a durometer. In an alternate embodiment the hardness is about 25 or less on the Shore A scale as measured with a durometer.

**Fig. 4** depicts a cross-sectional view of the flexible circuit array **10** being enveloped within an insulating material **11.** It shows how the edges of the material body **11** are lifted off due to the contracted radius at the edges. The electrode array **10** preferably also contains a fold A between the cable **12** and the electrode array **10.** The angle of the fold A relieves stress imparted to the array **10** by the cable **12.** The insulating material **11** further includes tack opening or attachment point **56** and a heel **26.** When the array is tacked to a retina, the tack forms a fulcrum like point where pressure of the tissue against the heel **26** is transmitted to the array portion of the array body **10.**

It should be noted that a fulcrum is a simplification of the complex forces affecting such an electrode array. First the array is not flat as in a typical lever and fulcrum. Further, the array is not rigid as in a typical lever and fulcrum. The tack force gradually decreases across the array surface moving away from the tack. There is a force of the retina pressing against nearly the entire array surface. The array curvature can not perfectly match the curvature of every retina. Thus some flexibility is necessary to achieve good electrode contact with the retina. Nevertheless, the concept of a fulcrum is a useful tool to illustrate the invention.

**Fig. 5** depicts a cross-sectional view of the flexible circuit array **10** being enveloped within an insulating material **11** with open electrodes **13** and the material **11** between the electrodes **13.**

**Fig. 6** depicts a cross-sectional view of the flexible circuit array **10** being enveloped within an insulating material **11** with open electrodes **13.** This is another embodiment wherein the electrodes **13** are not separated by the material **11** but the material **11** extends beyond the electrodes forming a window around the electrodes.

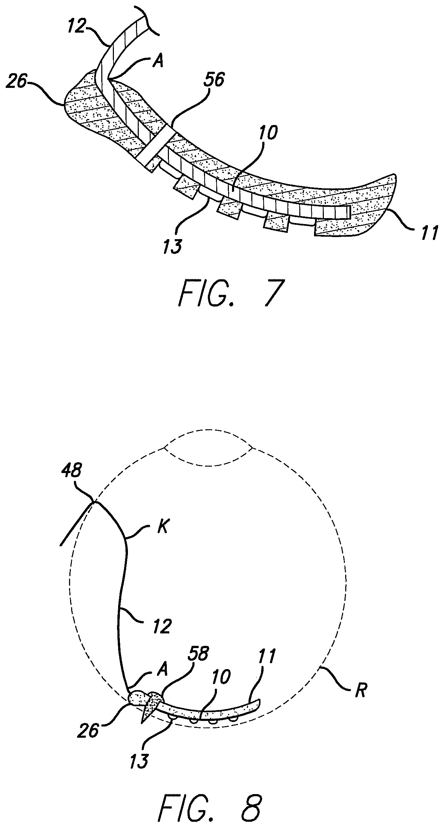

**Fig. 7** depicts a cross-sectional view of the flexible circuit array **10** being enveloped within an insulating material **11** in a spherical shape to match the curvature of the retina. The spherical shape is flattened at the heel **26** to exert pressure across the fulcrum like point caused by a tack placed through aperture attachment point **56.** The spherical shape is further altered at the edges of the array body to cause the array body to gently lift off, or retreat from, the retina without sharp edges.

**Fig. 8** depicts a cross-sectional view of the flexible circuit array **10** being enveloped within an insulating material **11** with electrodes **13** on the surface of the material **11** inside the eye, with a fold K in the flexible circuit cable **12,** and a fold A between the circuit electrode array **10** and the flexible circuit cable **12.** The material **11** and electrode array body **10** are in intimate contact with retina R. The surface of electrode array body **10** in contact with retina R is a curved surface with a similar radius compared to the spherical curvature of retina R to control pressure concentrations therein. Further, the decreasing radius of spherical curvature of material **11** near its edge forms edge relief that causes the edges of the body **11** to lift off the surface of retina R eliminating pressure concentrations at the edges. The edge of body **11** is rounded to reduce pressure and cutting of retina R. The heel **26** may be less spherical and causes pressure to be transferred beyond the tack **58** across the entire electrode array **10.** The heel **26** may be altered in curvature, hardness or thickness to achieve the desired pressure on the array surface.

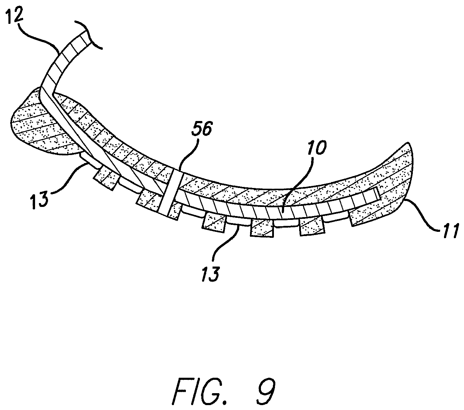

**Fig. 9** illustrates two alternate embodiments. **Fig. 9** depicts a cross-sectional view of the flexible circuit array **10** being enveloped within an insulating material **11** in a spherical shape to match the curvature of the retina. The spherical shape is flattened, but rather than having a thickened heel portion, the array is simply flatter than the curvature of the retina. The flattened portion exerts pressure across the fulcrum like point caused by a tack placed through aperture **56.** The spherical shape is further altered at the edges of the array body to cause the array body to gently lift off the retina without sharp edges.

A further alternate embodiment includes electrodes **13** on both sides of the tack aperture **56.** This provides a wider field of view.

The electric field produced by an electrode in which current is applied falls off with distance. Thus, it is advantageous to be as close to a target cell with a stimulating electrode as possible in order to minimize the injected charge required. It is advantageous to minimize injected charge for a number of reasons. First, the dynamic range of the possible stimulus will be much greater. Secondly, the battery life of a neural prosthesis in general (and a retinal prosthesis specifically) is largely driven by the total charge injected into the tissue.

Two types of electrode arrays have been tried in patients. The first type was a stiff array which applies a force that was designed to be greater than 10mmHg pressure. This array results in low thresholds therefore low electrical currents applied to the electrode were able to produce percepts that are stable over time. The retina does not degrade as previously thought. It has been found that a retina can be compressed by an electrode array in a stable way.

An array that is thinner and more flexible in general produces higher thresholds. In particular, portions of the array which may be lifted off the retina by some distance have even higher thresholds.

There are several ways that this pressure can be applied. First, the electrode array should be relatively stiff, especially if it is secured at one end, like by a tack. One way to make a flexible array made of silicone, polyimide or parylene more rigid is to secure a more rigid material such as metal wires either embedded in or on the array or higher duromer silicone to make the array into a high pressure contact structure. Also thickening the array will make it more rigid. The array should be more rigid than the surrounding tissue. Metal wires have the advantage that they can be shaped by the surgeon to accommodate a particular patient's retinal topography. It would also be advantageous if the tack could apply continuous force to press the array into the retina as by a spring tack, described in US Application 2006/0155288.

In one embodiment, the cable leading from the electronics package to the array head as disclosed in US Patents Nos. 5,935,155; 6,718,209; 7,228,181, and US Patent Application No. 2002/0111658, is preferably more flexible than the electrode array head, so the forces on the array are only produced by the spring tack and there is no tendency for the array to tip from one side to another due to the array cable.

In another embodiment, the cable is preferably made as rigid as the electrode array head, for example by the addition of metal wires or foil. If the cable has stiffening members that have a memory to them like metal as described above, the cable can then be positioned by the surgeon at the time of surgery to place the electrode array in the desired location and it will stay there.

In one embodiment, an increased pressure is preferably applied to just a portion of the electrode array body. For instance, a raised sealing feature, such as a silicone gasket, surrounding the area of the electrodes in an array, may be used to create a barrier to current flow. This has the effect of forcing more current through the retinal tissue and reducing the shunting of currents through the vitreous humor. The net result is a lowering of perceptual current stimulation thresholds. Lower thresholds result in greater dynamic range, longer electrode life, longer battery life and are generally believed to be safer to the tissue. Also, because the surface area is smaller than that of the entire electrode array, less total force can be applied to the array to create the desired localized high pressure which can seal the array to the tissue by forcing conformance between the shape of the retina and the shape of the electrode array. Because the pressure applied to the retina in this case is outside the active area of the device, even if retinal cells were damaged, they would be outside the area of interest.

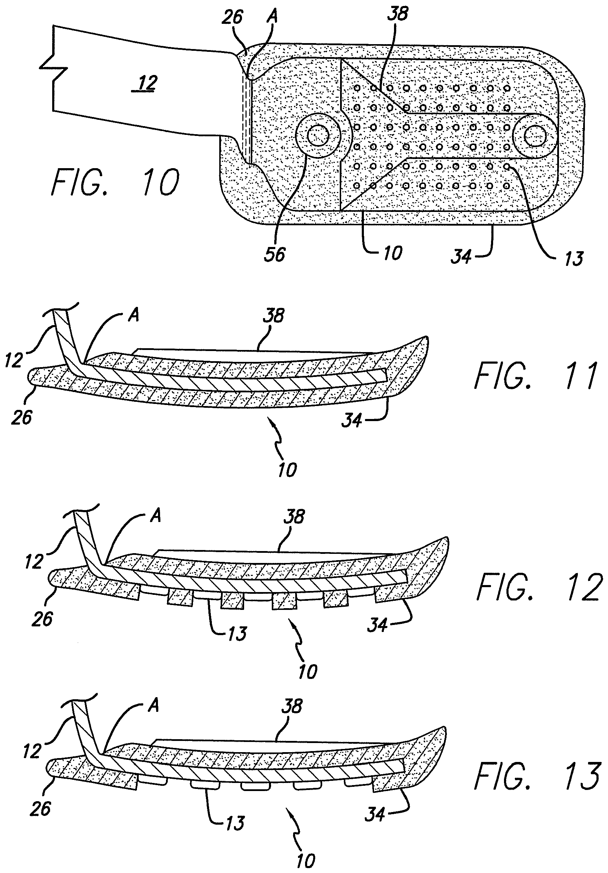

**Figure 10** depicts the top view of the flexible circuit array **10** being enveloped within a molded body **34.** The electrode array **10** is encased within the oval-shaped molded body **34,** a plurality of electrodes **13** made of a conductive material, such as platinum or one of its alloys, but that can be made of any conductive biocompatible material such as iridium, iridium oxide or titanium nitride. The electrode array **10** is enveloped within a molded body **34** that is preferably silicone. The molded body is extended in a heel **26**. The heel follows the contour of the array **10,** but may be stiffer to exert a force on the array field. "Oval-shaped" electrode array body means that the body may approximate either a square or a rectangle shape, but where the corners are rounded. This shape of an electrode array is described in the U.S. Patent Application No. 20020111658, entitled "Implantable retinal electrode array configuration for minimal retinal damage and method of reducing retinal stress" and No. 20020188282, entitled "Implantable drug delivery device" to Robert J. Greenberg et al.

The molded body **34** is made of a soft material that is compatible with the electrode array **10.** In a preferred embodiment the molded body **34** made of silicone having hardness of about 50 or less on the Shore A scale as measured with a durometer. In an alternate embodiment the hardness is about 25 or less on the Shore A scale as measured with a durometer.

**Figure 11** depicts a cross-sectional view of the flexible circuit array **10** being enveloped within the molded body **34.** It shows how the edges of the molded body **34** are lifted off due to the contracted radius at the edges. The electrode array **10** preferably also contains a fold A between the cable **12** and the electrode array **10.** The angle of the fold A relieves torque applied to the array **10** by the cable **12.**

**Figure 12** depicts a cross-sectional view of the flexible circuit array **10** being enveloped within a molded body **34** with open electrodes **13** and the molded body **34** between the electrodes **13.**

**Figure 13** depicts a cross-sectional view of the flexible circuit array **10** being enveloped within the molded body **34** with open electrodes **13.** This is another embodiment wherein the electrodes **13** are not separated by the molded body **34.** This may allow closer contact with the neural tissue.

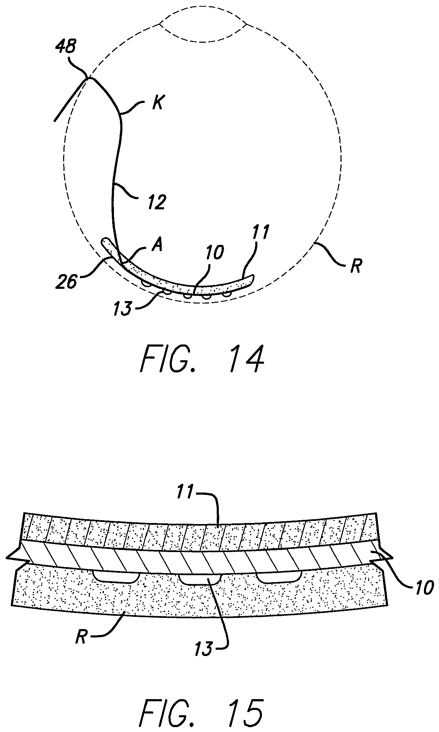

**Figure 14** depicts a cross-sectional view of the flexible circuit array **10** being enveloped within the molded body **34** with electrodes **13** on the surface of the molded body **34** inside the eye with an angle K in the fold of the flexible circuit cable **12** and a fold A between the circuit electrode array **10** and the flexible circuit cable **12.** The molded body **34** and electrode array body **10** are in intimate contact with retina R. The surface of electrode array body **10** in contact with retina R is a curved surface with a similar radius compared to the spherical curvature of retina R to minimize pressure concentrations therein. Further, the decreasing radius of spherical curvature of the molded body **34** near its edge forms edge relief that causes the edges of the molded body **34** to lift off the surface of retina R eliminating pressure concentrations at the edges. The edge of molded body **34** is rounded to reduce pressure and cutting of retina R.

**Figure 15** shows a part of the Figure 14 enlarged showing the electrode array **10** and the electrodes **13** enveloped by the molded body **34,** preferably silicone in intimate contact with the retina R.

The electrode array **10** embedded in or enveloped by the molded body **34** can be preferably produced through curing the silicone in a mold around the polyimide array **10.** The molded body **34** has a shape with a decreasing radius at the edges so that the edges of the molded body **34** lift off from the retina R.

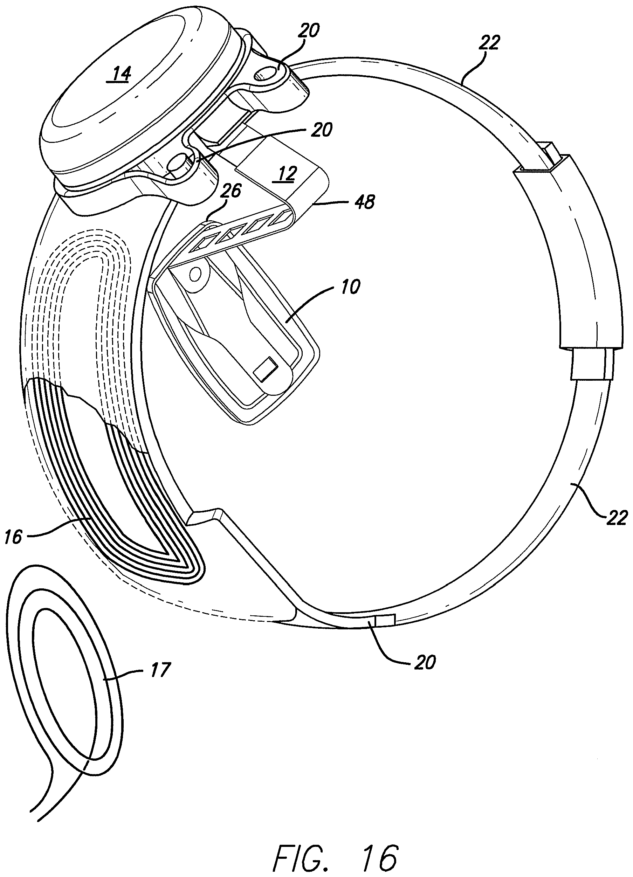

**Figure 16** shows a perspective view of the implanted portion of the preferred retinal prosthesis. A flexible circuit **1** includes a flexible circuit electrode array **10** which is mounted by a retinal tack (not shown) or similar means to the epiretinal surface. The flexible circuit electrode array **10** is electrically coupled by a flexible circuit cable **12,** which pierces the sclera and is electrically coupled to an electronics package **14,** external to the sclera.

The electronics package **14** is electrically coupled to a secondary inductive coil **16.** Preferably the secondary inductive coil **16** is made from wound wire. Alternatively, the secondary inductive coil **16** may be made from a flexible circuit polymer sandwich with wire traces deposited between layers of flexible circuit polymer. The secondary inductive coil receives power and data from a primary inductive coil **17,** which is external to the body. The electronics package **14** and secondary inductive coil **16** are held together by the molded body **18.** The molded body **18** holds the electronics package **14** and secondary inductive coil **16** end to end. The secondary inductive coil **16** is placed around the electronics package **14** in the molded body **18.** The molded body **18** holds the secondary inductive coil **16** and electronics package **14** in the end to end orientation and minimizes the thickness or height above the sclera of the entire device. The molded body **18** may also include suture tabs **20.** The molded body **18** narrows to form a strap **22** which surrounds the sclera and holds the molded body **18,** secondary inductive coil **16,** and electronics package **14** in place. The molded body **18,** suture tabs **20** and strap **22** are preferably an integrated unit made of silicone elastomer. Silicone elastomer can be formed in a pre-curved shape to match the curvature of a typical sclera. However, silicone remains flexible enough to accommodate implantation and to adapt to variations in the curvature of an individual sclera. The secondary inductive coil **16** and molded body **18** are preferably oval shaped. A strap **22** can better support an oval shaped coil. It should be noted that the entire implant is attached to and supported by the sclera. An eye moves constantly. The eye moves to scan a scene and also has a jitter motion to improve acuity. Even though such motion is useless in the blind, it often continues long after a person has lost their sight. By placing the device under the rectus muscles with the electronics package in an area of fatty tissue between the rectus muscles, eye motion does not cause any flexing which might fatigue, and eventually damage, the device.

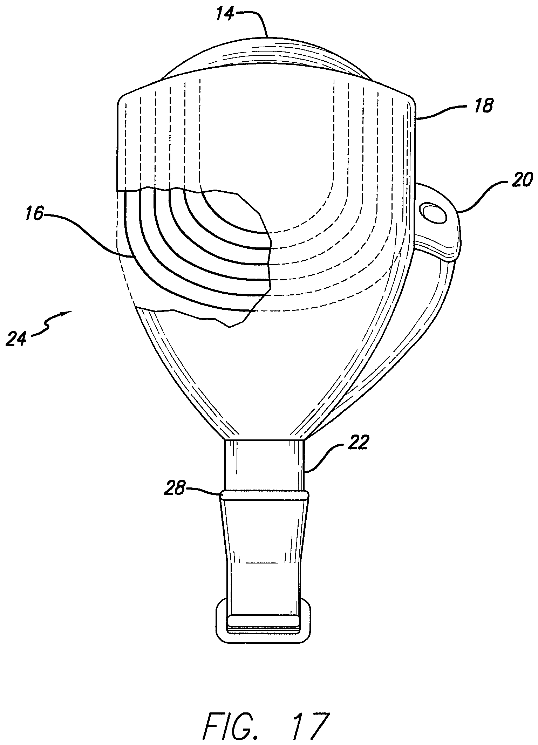

**Figure 17** shows a side view of the implanted portion of the retinal prosthesis, in particular, emphasizing the fan tail **24.** When implanting the retinal prosthesis, it is necessary to pass the strap **22** under the eye muscles to surround the sclera. The secondary inductive coil **16** and molded body **18** must also follow the strap **22** under the lateral rectus muscle on the side of the sclera. The implanted portion of the retinal prosthesis is very delicate. It is easy to tear the molded body **18** or break wires in the secondary inductive coil **16.** In order to allow the molded body **18** to slide smoothly under the lateral rectus muscle, the molded body **18** is shaped in the form of a fan tail **24** on the end opposite the electronics package **14.** The strap **22** further includes a hook **28** the aids the surgeon in passing the strap under the rectus muscles.

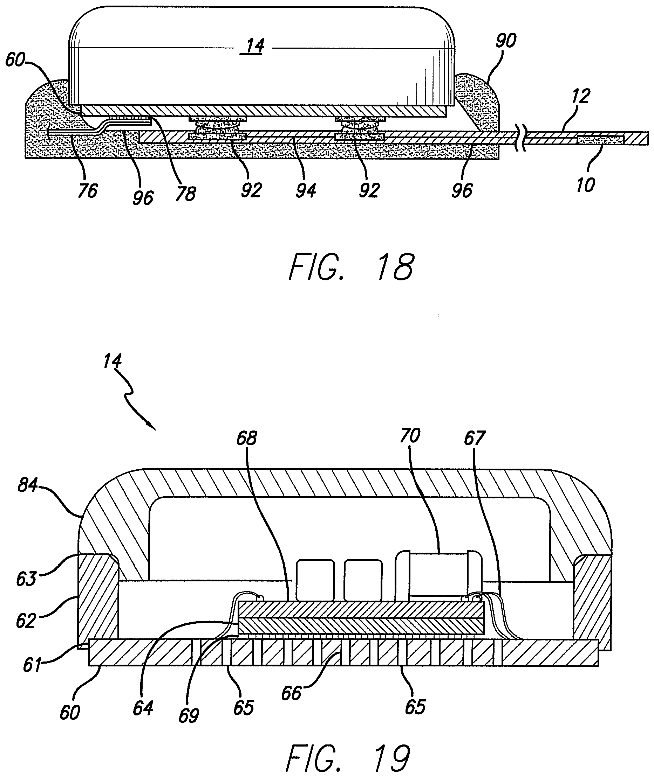

Referring to **figure 18****,** the flexible circuit **1,** includes platinum conductors **94** insulated from each other and the external environment by a biocompatible dielectric polymer **96,** preferably polyimide. One end of the array contains exposed electrode sites that are placed in close proximity to the retinal surface **10.** The other end contains bond pads **92** that permit electrical connection to the electronics package **14.** The electronic package **14** is attached to the flexible circuit **1** using a flip-chip bumping process, and epoxy underfilled. In the flip-chip bumping process, bumps containing conductive adhesive placed on bond pads **92** and bumps containing conductive adhesive placed on the electronic package **14** are aligned and melted to build a conductive connection between the bond pads **92** and the electronic package **14.** Leads **76** for the secondary inductive coil **16** are attached to gold pads **78** on the ceramic substrate **60** using thermal compression bonding, and are then covered in epoxy. The electrode array cable **12** is laser welded to the assembly junction and underfilled with epoxy. The junction of the secondary inductive coil **16,** array **1,** and electronic package **14** are encapsulated with a silicone overmold **90** that connects them together mechanically. When assembled, the hermetic electronics package **14** sits about 3mm away from the end of the secondary inductive coil.

Since the implant device is implanted just under the conjunctiva it is possible to irritate or even erode through the conjunctiva. Eroding through the conjunctiva leaves the body open to infection. We can do several things to lessen the likelihood of conjunctiva irritation or erosion. First, it is important to keep the over all thickness of the implant to a minimum. Even though it is advantageous to mount both the electronics package **14** and the secondary inductive coil **16** on the lateral side of the sclera, the electronics package **14** is mounted higher than, but not covering, the secondary inductive coil **16.** In other words the thickness of the secondary inductive coil **16** and electronics package should not be cumulative.

It is also advantageous to place protective material between the implant device and the conjunctiva. This is particularly important at the scleratomy, where the thin film electrode array cable **12** penetrates the sclera. The thin film electrode array cable **12** must penetrate the sclera through the pars plana, not the retina. The scleratomy is, therefore, the point where the device comes closest to the conjunctiva. The protective material can be provided as a flap attached to the implant device or a separate piece placed by the surgeon at the time of implantation. Further material over the scleratomy will promote healing and sealing of the scleratomy. Suitable materials include DACRON^{®}, TEFLON^{®}, GORETEX^{®} (ePTFE), TUTOPLAS^{®} (sterilized sclera), MERSILENE^{®} (polyester) or silicone.

Referring to **figure 19****,** the package **14** contains a ceramic substrate **60,** with metalized vias **65** and thin-film metallization **66.** The package **14** contains a metal case wall **62** which is connected to the ceramic substrate **60** by braze joint **61.** On the ceramic substrate **60** an underfill **69** is applied. On the underfill **69** an integrated circuit chip **64** is positioned. On the integrated circuit chip **64** a ceramic hybrid substrate **68** is positioned. On the ceramic hybrid substrate **68** passives **70** are placed. Wirebonds **67** are leading from the ceramic substrate **60** to the ceramic hybrid substrate **68.** A metal lid **84** is connected to the metal case wall **62** by laser welded joint **63** whereby the package **14** is sealed.

Accordingly, what has been shown is an improved method making a neural electrode array and improved method of stimulating neural tissue. While the invention has been described by means of specific embodiments and applications thereof, it is understood that numerous modifications and variations could be made thereto by those skilled in the art without departing from the scope of the invention. It is therefore to be understood that within the scope of the claims, the invention may be practiced otherwise than as specifically described herein.

## Claims

1. An electrode array (10) for neural stimulation comprising:
an attachment point (56) suitable to be attached to tissue; a plurality of electrodes (13) defining an electrode region suitable to make contact with neural tissue, on one side of said attachment point;
a cable (12) suitable to supply said plurality of electrodes; and
a heel (26) on an opposite side of said attachment point from the electrode region forming a solid body with said electrode region, **characterized in that**:
the heel is adapted to deviate from the primary curvature of the electrode region to be suitable to make contact with tissue, causing the electrode region to apply pressure on the neural tissue.

2. The electrode array according to claim 1, wherein
the attachment point is adapted to form an anchoring mechanism interfaced between said electrode region and said heel; and said solid body deflects under the application of applied anchoring force and said deflection is controlled by selection of curvature, stiffness and surface profile of the heel.

3. The electrode array according to claim 1, wherein the neural tissue is the neural tissue of the retina; and the electrode region is curved to approximate the curvature of the retina.

4. The electrode array according to claim 1, 2 or 3, wherein electrodes are in only said electrode region.

5. The electrode array according to claim 1, 2, or 3, wherein said heel is less curved than said electrode region.

6. The electrode array according to claim 1, 2 or 3, wherein said heel is thicker than said electrode region.

## Patentansprüche

1. Elektrodenanordnung (10) zur Nervenstimulierung, umfassend:
einen Befestigungspunkt (56), der geeignet ist, um auf Gewebe befestigt zu werden;
eine Vielzahl von Elektroden (13), die eine Elektrodenregion definieren, die geeignet ist, um auf einer Seite des Befestigungspunkts Nervengewebe zu berühren;
ein Kabel (12), das geeignet ist, um die Vielzahl von Elektroden zu versorgen; und
einen Absatz (26) auf einer der Elektrodenregion entgegengesetzten Seite des Befestigungspunkts, der einen Festkörper mit der Elektrodenregion ausbildet, **dadurch gekennzeichnet, dass**:
der Absatz angepasst ist, um von der primären Krümmung der Elektrodenregion abzuweichen, um Gewebe berühren zu können, was bewirkt, dass die Elektrodenregion Druck auf das Nervengewebe ausübt.

2. Elektrodenanordnung nach Anspruch 1, wobei der Befestigungspunkt geeignet ist, um einen Ankermechanismus auszubilden, der eine Grenzfläche zwischen der Elektrodenregion und dem Absatz ist; und wobei der Festkörper sich unter der Ausübung von beaufschlagter Ankerkraft durchbiegt und wobei die Durchbiegung durch Auswahl von Krümmung, Steifigkeit und Oberflächenprofil des Absatzes gesteuert ist.

3. Elektrodenanordnung nach Anspruch 1, wobei das Nervengewebe das Nervengewebe der Netzhaut ist; und wobei die Elektrodenregion ungefähr der Krümmung der Netzhaut entsprechend gekrümmt ist.

4. Elektrodenanordnung nach Anspruch 1, 2 oder 3, wobei sich nur in der genannten Elektrodenregion Elektroden befinden.

5. Elektrodenanordnung nach Anspruch 1, 2 oder 3, wobei der Absatz weniger gekrümmt ist als die Elektrodenregion.

6. Elektrodenanordnung nach Anspruch 1, 2 oder 3, wobei der Absatz dicker ist als die Elektrodenregion.

## Revendications

1. Réseau d'électrodes (10) pour la stimulation neurale comprenant :
un point de fixation (56) conçu pour être fixé au tissu ;
une pluralité d'électrodes (13) définissant une région d'électrodes conçues pour entrer en contact avec du tissu neural, sur un côté dudit point de fixation ;
un câble (12) conçu pour alimenter ladite pluralité d'électrodes ;
un talon (26) sur un côté opposé dudit point de fixation de la région d'électrodes formant un corps solide avec ladite région d'électrodes,
**caractérisé en ce que** :
le talon sert est adapté pour dévier de la courbure principale de la région d'électrodes pour pouvoir entrer en contact avec le tissu, amenant la région d'électrodes à appliquer une pression sur le tissu neural.

2. Réseau d'électrodes selon la revendication 1, dans lequel
le point de fixation est adapté pour former un mécanisme d'ancrage en interface entre ladite région d'électrodes et ledit talon ; et ledit corps solide dévie sous l'application de la force d'ancrage appliquée et ladite déviation est contrôlée par le choix d'une courbure, d'une rigidité et d'un profil de surface du talon.

3. Réseau d'électrodes selon la revendication 1, dans lequel le tissu neural est le tissu neural de la rétine ; et la région d'électrodes est incurvée pour s'approcher de la courbure de la rétine.

4. Réseau d'électrodes selon la revendication 1, 2 ou 3 dans lequel les électrodes ne sont que dans ladite région d'électrodes.

5. Réseau d'électrodes selon la revendication 1, 2 ou 3, dans lequel ledit talon est moins incurvé que ladite région d'électrodes.

6. Réseau d'électrodes selon la revendication 1, 2 ou 3, dans lequel ledit talon est plus épais que ladite région d'électrodes.
